Europäisches Patentamt
European Patent Office
Office européen des brevets

(11) Veröffentlichungsnummer: **0 185 281**
**A2**

# (12) EUROPÄISCHE PATENTANMELDUNG

(21) Anmeldenummer: 85115602.6

(22) Anmeldetag: 07.12.85

(51) Int. Cl.[4]: **A 61 K 37/02**

(30) Priorität: 17.12.84 DE 3445933

(43) Veröffentlichungstag der Anmeldung: 25.06.86 Patentblatt 86/26

(84) Benannte Vertragsstaaten: AT BE CH DE FR GB IT LI NL SE

(71) Anmelder: Röhm GmbH
Kirschenallee Postfach 4242
D-6100 Darmstadt 1(DE)

(72) Erfinder: Kramer, Herbert J., Prof. Dr.
Augustastrasse 67
D-5300 Bonn 2(DE)

(72) Erfinder: Lehmann, Klaus, Dr.
Schillerstrasse 85
D-6101 Rossdorf(DE)

(72) Erfinder: Liddiard, Colin, Dr.
Ginsterweg 17
D-6108 Weiterstadt(DE)

## (54) Arzneimittel mit diuretischer Wirksamkeit.

(57) Die Erfindung betrifft Arzneimittel mit diuretischer Wirksamkeit, die als Wirkstoff ein Peptid der Formal I

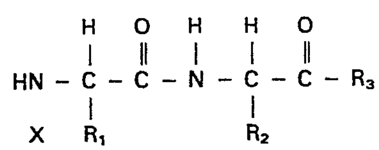

I

worin a) $R_1$ und $R_2$ für Wasserstoff und $R_3$ für $-OH$ oder für einen Rest

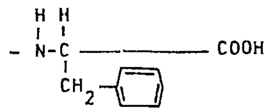

oder einen Rest

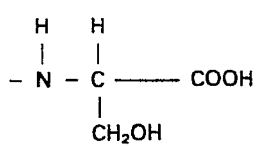

oder einen Rest Y oder
worin b) $R_1$ für Wasserstoff, $R_2$ für $CH_2OH$ oder 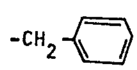 und $R_3$ für $-OH$ oder einen Rest Y oder

worin c) $R_1$ für einen Rest $-CH_2-OH$ und $R_2$ für Wasserstoff oder einen Rest

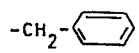

und $R_3$ für $-OH$ oder einen Rest Y oder worin d) $R_1$ für einen Rest

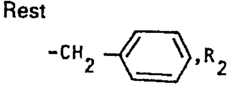

für Wasserstoff oder für einen Rest $-CH_2OH$ und $R_3$ für $-OH$ oder einen Rest

$$\overset{H}{-N}-CH_2-COOH$$

oder einen Rest Y stehen und worin X für Wasserstoff, Methyl, einen Prolylrest oder eine N-Schutzgruppe und Y für $-NH_2$, $-OR_4$, worin $R_4$ für einen gegebenenfalls verzweigten, gegebenenfalls cyclischen Alkylrest mit 1 bis 8 Kohlenstoffatomen, einen Benzyl- oder Phenylrest oder einen Rest

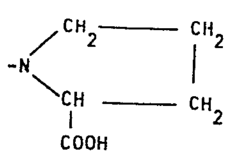

./...

EP 0 185 281 A2

steht,
oder solche Vorstufen, die unter physiologischen Bedingungen Peptide der Formel I bilden
enthalten.

# Arzneimittel mit diuretischer Wirksamkeit

## Gebiet der Erfindung

Die Erfindung betrifft Arzneimittel mit diuretischer. speziell mit natriuretischer Wirksamkeit. deren Wirkstoffe niedermolekulare Peptide darstellen.

Die Arzneimittel eignen sich insbesondere zur Anwendung bei Krankheitsbildern, wo vermehrte Harnausscheidung therapeutisch erwünscht ist. Hervorzuheben ist, daß vorwiegend bis ausschließlich die Ausscheidung von Natriumsalzen, speziell Kochsalz, nicht dagegen von Kaliumsalzen forciert wird. Die Arzneimittel eignen sich vor allem zur parenteralen, insbesondere zur intravenösen Verabreichung.

## Stand der Technik

Als Diuretika werden allgemein Substanzen bezeichnet, die eine vermehrte Harnausscheidung bewirken.
Die therapeutisch gebräuchlichen Diuretika lassen sich beispielsweise vom Wirkungstyp bzw. soweit bekannt, vom Wirkungsort bzw. Wirkungsmechanismus und/oder von ihren chemischen Strukturen her klassifizieren.

Gebräuchliche Klassifikationen unterscheiden beispielsweise osmotische Diuretika, Carboanhydrasehemmer. Thiazid-Diuretika. Schleifendiuretika, kaliumsparende Diuretika und in keine dieser Gruppe fallende Vertreter, wie z.B. die Xanthine (vgl. Kirk-Othmer, 3rd. Ed., Vol. 8, 1 - 33, John Wiley, 1979). Es handelt sich dabei durchweg um körperfremde Stoffe, wobei aromatische bzw. heterocyclische Strukturen einen bedeutenden Anteil ausmachen.

Diese körperfremden Wirkstoffe besitzen in der Regel Nebenwirkungen, die, insbesondere bei Dauertherapie, nicht zu vernachlässigen sind. So können z.B. Störungen im Elektrolythaushalt als Folge der Anwendung der Diuretika auftreten, vor allem Hypokaliämie. Dieser Zustand des subnormalen Kaliumspiegels im Serum kann zum Teil durch Kaliumzufuhr, wirksamer aber durch die gleichzeitige Verabreichung kaliumretinierender Diuretika (Spironolacton, Triamteren, Amilorid) vermieden werden. Seit über 25 Jahren wird die Existenz eines natriuretischen Hormons vermutet, das im Organismus die Kochsalzausscheidung fördert. Bis jetzt konnte ein solches natriuretisches Hormon nicht eindeutig identifiziert werden, jedoch legen die Ergebnisse von Aktivitätsbestimmungen an Modellsystemen mit Fraktionen aus Serum und Harn von mit Kochsalz behandelten Ratten, Hunden und gesunden Versuchspersonen nahe, daß das vermutete natriuretische Hormon eine relativ niedermolekulare Substanz sein muß (vgl. H.J.Kramer et al., "Nachweis und Charakterisierung natriuretischer Faktoren in Plasma und Urin", Abstract in "Nieren- und Hochdruckkrankheiten" 8, 236, 1979). Als Wirkmechanismus des Natriuretischen Hormons (NH) wird die Inhibierung der $Na^+$-$K^+$-abhängigen ATP-ase angenommen, die den transepithelialen Natrium-Transport beeinflußt (H.C. Gonick et al. in Clin.Sci.Mol.Med. 53, 329 - 334, 1977). Die Struktur dieses vermuteten natriuretischen Hormons (NH) muß weiterhin als unbekannt gelten.

Hormonwirkung haben zahlreiche Oligopeptide, die im Zentralnervensystem vorgefunden wurden. Sie üben z.B. Einfluß auf den gastrointestinalen Bereich oder den Verhaltensbereich aus und sie wirken modifizierend auf gewisse Drüsenfunktionen ein. Kleine Peptidhormone sind z.B. das Thyroliberin (Thyrotropin-Releasing-Factor = TRF), welches die Sekretion des Thyroidstimulierenden Hormons bei Ratte und Maus stimuliert, sowie die Enkephaline, die nur aus dem Hypophysenanhang isoliert werden konnten und die morphinomimetische Wirkung ausüben.

Man schreibt der als "Brain Oligopeptides" zusammengefaßten Gruppe die Rolle von extracellulären Boten-Substanzen zu, die in der Informationsübertragung und in der Neuroregulation vermutlich eine wichtige Rolle spielen (vgl. Kirk-Othmer, 3rd Ed. Vol. 12, pg 603 - 617, J. Wiley, 1980).
Das regulatorische Geschehen spielt sich nach geltenden Vorstellungen unter Einschaltung spezifischer Rezeptoren ab. Das Zusammenwirken zwischen Botensubstanz und Rezeptor und der Wirkmechanismus der Regulation sind nur in einzelnen Fällen wirklich transparent geworden. Von einer gezielten Modifizierung im Sinne einer therapeutischen Beeinflussung des Stoffwechsels kann ebenfalls nur in relativ wenigen Fällen die Rede sein.

## Aufgabe

Obwohl der therapeutischen Praxis pharmazeutische Prinzipien zur Verfügung stehen, mit denen sich eine auf den Einzelfall ausgerichtete Diuresetherapie durchführen läßt, so besteht dennoch im individuellen Falle Unsicherheit darüber, wie der Organismus auf das Medikament ansprechen, welche Nebenwirkungen auftreten und wie er auf Dauermedikamentierung reagieren würde.

Als Indikationen einer diuretischen Therapie sind anzusehen:

1. Generalisiertes Ödemleiden mit erhöhtem Natriumbestand des Organismus (chronische Herzinsuffizienz, Linksinsuffizienz mit Lungenstauung und Lungenödem, Lebercirrhose mit Ascites, Nierenkrankheiten)

2. Lokale Ödeme.

3. Diuretika-Indikationen ohne Ödem (arterielle Hypertension, Vergiftungen) und sonstige Indikationen wie Diabetes insipidus, Glaukom, Elimination von Calcium, Kalium, Magnesium, Retention von Kalium und Magnesium, idiopathisches Ödem (vgl. D.P. Mertz in "Arztblatt Baden-Württemberg" (3) 1974, wo auch die herausgebildeten therapeutischen Schemata beschrieben werden).

Es bestand nach wie vor Bedarf an Diuretika, die bei ausreichender diuretischer Wirksamkeit eine möglichst geringe Belastung des Organismus, vor allem im Hinblick auf Nebenwirkungen, mit sich bringen sollten. Die diuretisch wirksamen Prinzipien sollten nach Möglichkeit keine Probleme hinsichtlich Metabolisierung aufweisen. Außerdem sollten sie leicht zugänglich und handhabbar und in möglichst stabile Applikationsformen überführbar sein.

Schließlich bestand ein Bedürfnis nach einem spezifisch natriuretischen Wirkstoff, d.h. der Kalium- und Magnesiumstoffwechsel sollte durch die Diurese möglichst wenig in Mitleidenschaft gezogen werden.

<u>Lösung</u>

Es wurde gefunden, daß bestimmte Peptide als Wirkstoffe bei der Therapie von durch Diurese positiv beeinflußbaren Krankheiten besonders gut geeignet sind. Bei den diuretisch beeinflußbaren Krankheiten handelt es sich in erster Linie um die oben (s.Aufgabe) genannten.

Bei den erfindungsgemäß als diuretische Wirkstoffe anzuwendenden Peptiden seien diejenigen der Formel I

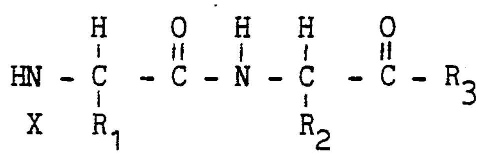

I

worin a) $R_1$ und $R_2$ für Wasserstoff und $R_3$ für -OH oder für einen Rest

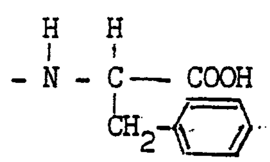

oder einen Rest

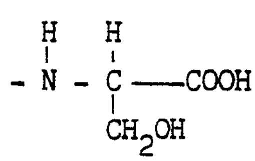

oder einen Rest Y, oder

worin b) $R_1$ für Wasserstoff, $R_2$ für $CH_2OH$ oder $-CH_2-$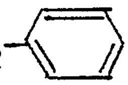 und $R_3$ für -OH oder einen Rest Y oder

worin c) $R_1$ für einen Rest $-CH_2-OH$ und $R_2$ für Wasserstoff oder einen Rest $-CH_2-$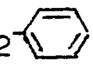 und $R_3$ für OH oder einen Rest Y

worin d) $R_1$ für einen Rest $-CH_2-$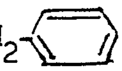 $R_2$ für Wasserstoff oder für einen Rest $-CH_2OH$ und $R_3$ für -OH oder für einen Rest $\overset{H}{N}-CH_2COOH$ oder einen Rest Y stehen, und worin X für Wasserstoff, Methyl einen Prolylrest oder eine N-Schutzgruppe und Y für $-NH_2$, $-OR_4$, worin $-R_4$ für einen gegebenenfalls verzweigten, gegebenenfalls cyclischen Alkylrest mit 1 bis 8 Kohlenstoffatomen, einen Benzyl- oder Phenylrest

oder einen Rest

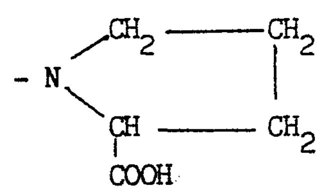

steht,

sowie solche Vorstufen, die unter physiologischen Bedingungen Peptide der Formel I bilden, genannt.

Unter einer N-Schutzgruppe seien an sich übliche Schutzgruppen (vgl. R.A. Boissonas in Advances in Organic Chemistry Vol.3, pp 159 - 183, Interscience, 1963) verstanden, insbesondere N-Acylgruppen, wie Carboxyestergruppen, z.B. die gegebenenfalls mit Nitro-, Chloro- oder Phenylazoresten substituierte Carbobenzyloxygruppe, die Carbotert-butyloxy-, Carbocyclo-pentyloxy-, Fettsäure, wie die Formyl-, Trifluoracetyl-, aromatische Säure, wie Benzoyl-, Phthaloyl-, Tosyl-Gruppe.

Als Rest $R_4$ seien insbesondere der Methyl-, Ethyl-, tert.-Butyl als cyclische Reste $R_4$ besonders solche mit 5 bis 8 Ringkohlenstoffatomen, insbesondere Cyclohexyl genannt.
Die Peptide der Formel I sind, wie durch die Formeldarstellung zu erkennen, aus den Aminosäuren Glycin (Gly), Phenylalanin (Phe) und Serin (Ser) aufgebaut. Zur therapeutischen Anwendung sind nicht nur Peptide aus den natürlich vorkommenden L-Aminosäuren geeignet, sondern auch die Razemate bzw. die aus den entsprechenden D-Aminosäuren gebildeten. Es handelt sich in der Regel um kleine Peptide mit drei oder zwei Aminosäuren als Bausteinen, wobei entweder mindestens ein Gly im Peptid vorhanden sein soll, oder das Peptid aus unter sich verschiedenen Aminosäuren aufgebaut sein soll.

Bevorzugt sind solche Vertreter, bei denen Glycin die N-terminale oder die C-terminale Position oder beide einnimmt. Insgesamt sind die zwei Glycinreste enthaltenden Peptide bevorzugt, besonders diejenigen mit zwei miteinander verknüpften Glycinresten, wovon der eine C-terminal steht. Besonders genannt seien die Peptide

L-Phe-Gly-Gly
Gly-Gly
Gly-Gly-L-Phe
Gly-L-Phe
L-Phe-Gly
L-Ser-Gly
Gly-L-Ser
L-Phe-L-Ser.

Die erfindungsgemäß anwendbaren Peptide der Formel I sind in der Regel uneingeschränkt wasserlöslich.

Die erfindungsgemäß wirksamen Peptide der Formel I sind verhältnismäßig einfach aufgebaute Verbindungen, die überwiegend bekannt sind. Ihre Herstellung ist bekannt, bzw. sie kann in an sich bekannter Weise vorgenommen werden. (Vgl. Ullmanns Encyclopädie der technischen Chemie, 4. Auflage, Bd. 19, 540 - 558, Verlag Chemie.) Auch die entsprechenden D-Aminosäuren, die als Bausteine in Frage kommen, sind an sich bekannt.

Bei der Synthese wird im Falle des Vorliegens von Serin die freie Hydroxygruppe als tert.-Butylether geschützt. Die terminale Carbonsäure wird als Ester, vorzugsweise als Methylester geschützt.

Ebenfalls wird die terminale Aminogruppe in an sich bekannter Weise geschützt (vgl. R.A. Boissonas in "Advances in Organic Chemistry" Vol. 3, pp 159 - 190 Ed. R. Raphael et al. Interscience Publishers, New York, 1963) beispielsweise mittels einer Carbobenzoxygruppe (Z). Die Verknüpfung der Bausteine geschieht vorteilhaft über aktivierte Derivate, beispielsweise gemischte Anhydride mit z.B. Chlorameisensäureester . Alternativ kann die Verknüpfung auch mit Carbodiimiden als aktivierenden Agentien vorgenommen werden, z.B. mit Dicyclohexylcarbodiimid, gegebenenfalls unter Zusatz von racemisierungssenkenden Zusätzen wie 1-Hydroxybenzotriazol, 3-Hydroxy-4-oxo-3,4-dihydro-1,2,3-benzotriazin, N-Hydroxysuccinimid. Die Abspaltung der Carbobenzoxyschutzgruppe (Z) erfolgt zweckmäßig durch katalytische Hydrierung. Man erhält so ein Peptid mit freier NH-Gruppe, die erneut mit einer N-geschützten Aminosäure zur Reaktion gebracht werden kann.

Die Z-Gruppe wird durch katalytische Hydrierung, vorwiegend mit

Palladium-Katalysatoren bei Raumtemperatur und Normaldruck abgespalten. Die tert.-Butylgruppe als Schutzgruppe der Hydroxygruppe im Serin kann durch Säuresolvolyse (Trifluoressigsäure) abgespalten werden (vgl. Houben-Weyl, Methoden der Organischen Chemie, 4. Auflage, Bd. 15, Thieme-Verlag, Stuttgart 1974). Die Estergruppen wie die Ethyl- oder Methylestergruppe, lassen sich hydrolytisch, insbesondere alkalisch, abspalten und ergeben so das freie Peptid. Benzyl- und Nitrobenzylester können durch katalytische Hydrierung abgespalten werden.

Im allgemeinen sind die erfindungsgemäßen Arzneimittel eher auf eine parenterale als eine orale Verabreichung ausgerichtet, jedoch sei auch die letztere nicht ausgeschlossen, insbesondere, wenn durch geeignete Umhüllung die Magen-Darm-Passage ohne Verlust der Aktivität gewährleistet ist. Sie eignen sich zur intravenösen, subkutanen oder intramuskulären Verabreichung. Möglicherweise ist auch eine Resorption durch die Nasenschleimhäute für die Therapie nutzbar zu machen.

Die erfindungsgemäßen Arzneimittel kommen in der Regel in Dosierungen ab 250 µg pro kg Körpergewicht zur Anwendung. Im allgemeinen liegen die Dosierungen bei 250 bis 2500 µg/kg als Richtwert bei ca. 500 µg/kg.

Die erfindungsgemäßen Wirkstoffe kommen, wie bereits ausgeführt, vorteilhaft in Form von Lösungen zur intravenösen Verabreichung zur Anwendung, beispielsweise in den für intravenöse Verabreichung geeigneten wäßrigen Lösungen, wobei die Lösungen wie üblich steril, schwebestofffrei haltbar und in größeren Mengen als 10 ml pyrogenfrei sein müssen.

Im Interesse der Verträglichkeit wird die Trägerflüssigkeit zweckmäßig in üblicher Weise dem Blutserum angeglichen; man wird daher auf Isotonie und Isohydrie mit dem Blutserum achten. (Vgl. F.Gstirner, Einführung in die Verfahrenstechnik der Arzneiformung, 5. Auflage, S. 255 - 307, Wissenschaftliche Verlagsgesellschaft, 1973.)

Besonders günstig gestaltet sich die Verabreichung in Kombination mit parenteral eingeführten Ernährungslösungen. Bekanntlich werden bei Dauerinfusionen Lösungen zugeführt, die Gemische von (essentiellen) Aminosäuren enthalten. Diesen Dauerinfusionslösungen können die erfindungsgemäß anzuwendenden Peptide der Formel I mit Vorteil zugesetzt und als solche verabreicht werden.

Ein besonderer Wirkungsvorteil der erfindungsgemäßen, die Peptide der Formel I enthaltenden Arzneimittel ist, daß sie zwar natriuretisch wirken, d.h. eine vermehrte Natriumchlorid-Ausscheidung verglichen mit dem unbehandelten Zustand hervorrufen, ohne aber gleichzeitig eine wesentliche vermehrte Kaliumausscheidung zu bewirken, d.h. die Wirkstoffe der Formel I wirken spezifisch natriuretisch.

Von Interesse ist ferner eine Kombination der natriuretisch wirksamen Peptide der Formel I mit den sogenannten Calcium-Antagonisten.
Unter Calcium-Antagonisten seien die calcium-antagonistischen Hemmstoffe der elektromechanischen Koppelung verstanden (vgl. Ullmanns Encyclopädie der Technischen Chemie, 4. Auflage, Bd. 10 S. 639). Als Partner eignen sich beispielsweise das Verapamil,

Methoxyverapamil, Gallopamil, Nifedipin, Prenylamin, Fendilin, Diltiazem.

Die zu verwendenden Calcium-Antagonisten lassen sich zum Teil durch die allgemeine Formel II darstellen:

$$R_5(CH_2)_2 - \overset{R_7}{N} - R_6 \qquad II$$

worin $R_7$ Wasserstoff oder Methyl,

$R_5$ einen Rest $(C_6H_5)_2CH-$ oder einen Rest

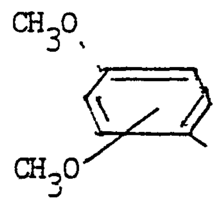

$R_6$ einen Rest $-CH(CH_3)-(CH_2)_n-C_6H_5$

worin n für 0 oder 1 steht oder einen Rest

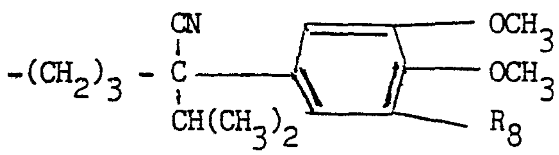

worin R für Wasserstoff oder eine Methoxygruppe steht, bedeuten, mit der Maßgabe, daß in ein- und demselben Molekül der allgemeinen Formel II nur entweder unsubstituierte Phenylreste und ein sekundäres Stickstoffatom ($R_7$ = Wasserstoff) oder aber durch Methoxygruppen substituierte Phenylreste und ein tertiäres Stickstoffatom ($R_7$ = Methyl) gleichzeitig vorhanden sein sollen, bedeuten oder/und die davon abgeleiteten, pharmakologisch vertretbaren Säureadditionssalze.

Die Dosierungen an den Calcium-Antagonisten als Komponente bei den erfindungsgemäßen Arzneimitteln liegen im Bereich 0,5 bis 20 mg/kg, vorzugsweise 5 bis 15 mg/kg. Sie können den Zubereitungen in an sich bekannter Weise zugefügt werden. Der Nachweis der diuretischen, speziell natriuretischen und antikaliuretischen Wirkung der Peptide der Formel I kann z.B. in Versuchen an Ratten erbracht werden.

Im vorliegenden Fall erfolgte die Bestimmung der natriuretischen Aktivität nach H.J. Kramer, C. Rietzel, D. Klingmüller und R. Düsing "Further studies on isolation and purification of a smaller molecular weight natriuretic hormone" in Lichardus, Schrier, Ponec "Hormonal Regulation of Sodium Excretion", Elsevier/North Holland, Amsterdam, 1980.

Versuchsanordnung

Als Versuchstiere werden weibliche Sprague-Dawley-Ratten mit einem Gewicht zwischen 206 bis 250 g verwendet. Vor dem Versuch werden die Ratten normaler Diät ausgesetzt und hatten freien Zugang zu Futter und Wasser. Am Versuchstag werden die Ratten mit Methohexital anästhetisiert und Katheter in die Hauptschlagader, die Halsvene und von dort abdomenwärts in die Blase geführt.

Die Tiere werden dann in Einzelkäfige, die auf einer Waage befestigt sind, verbracht. Anschließend gibt man i.v. 0,45 %ige Kochsalzlösung mit 2,5 % Fructose mit einer Geschwindigkeit von 33 - 62 µl/min, so daß das Körpergewicht konstant bleibt. Mit 15 minütigen Urinsammelperioden wird zwei Stunden nach der Erholung der Tiere begonnen und diese werden während der ganzen Untersuchung fortgeführt. Das zu testende Material der Formel I wird in der Infusionslösung, die Tris-HCl als Puffer (bei pH 7,4) enthält, gelöst. Bei den durch Natriurese gekennzeichneten Fraktionen werden 15-minütig Urinproben gesammelt, bis die Natriumausscheidung wieder die Basislinie erreicht hatte und die Infusionsgeschwindigkeit war so eingestellt, daß das Körpergewicht konstant bleibt. Der arterielle Blutdruck wird über das ganze Experiment hinweg mittels eines Druckmessers (Statham P-23-Db) verfolgt. Die Konzentrationen an Natrium und Kalium in Serum und Urin werden flammphotometrisch verfolgt. Die der mittleren Basislinie entsprechende Natriumausscheidung liegt bei 0,730 $\pm$ 0,028 µEqu/min.

Durchführung

Pro Ratte wurden 250 µg Substanz der Formel I verabreicht. Die in der folgenden Tabelle angegebenen Resultate wurden wie folgt gewonnen:

(1) stellt die Natrium-Ausscheidung bei unbehandelten Ratten (Mittelwerte aus 2 - 3 Einzelbestimmungen) in µMol $Na^+$ pro Minute dar,

(2) gibt die Natrium-Ausscheidung bei behandelten Ratten in µMol pro Minute pro mg verabreichter Substanz der Formel I an,

(3) Zeitraum der maximalen Wirkung in min. = $t_{max}$,

(4) stellt die Steigerung der Natriurese als Quotient

$$F = \frac{Na^+\text{-Ausscheidung nach Behandlung}}{Na^+\text{-Ausscheidung vor Behandlung}}$$ dar.

TABELLE

| Verbindung der Formel I | (1) $Na^+$-Ausscheidung | $Q = \frac{K^+}{Na^+}$ | (2) $Na^+$-Ausscheidung | $Q = \frac{K^+}{Na^+}$ | $t_{max}$ (min) | (3) F |
|---|---|---|---|---|---|---|
| Gly-Gly | 0,37 | 3,4 | 14,7 | 0,63 | 90 - 150 | 39,8 |
| L-Phe-Gly-Gly | 0,85 | 2,0 | 19,2 | 0,53 | 60 - 150 | 22,6 |
| Gly-Gly-L-Phe | 0,67 | 1,8 | 3,2 | 1,6 | 45 - 150 | 4,8 |
| L-Phe-L-Ser | 0,45 | 2,2 | 9,8 | 1,0 | 135 - 165 | 21,8 |
| Gly-L-Phe | 0,50 | - | 13,6 | - | 120 - 150 | 27,2 |
| L-Phe-Gly | 1,35 | - | 21,2 | - | 45 - 60 | 15,7 |
| L-Ser-Gly | 0,75 | - | 16,2 | - | 70 - 80 | 21,6 |
| Gly-L-Ser | 1,30 | - | 20,9 | - | 45 - 60 | 16,1 |

Ergebnis:

Die erfindungsgemäß behandelten Tiere zeigten eine erheblich angestiegene Natriuse nach Verabreichung der Wirkstoffe der Formel I.

Vergleichbare Resultate erhält man auch mit den Verbindungen L-Ser-Gly-Gly; L-Ser-L-Phe; Gly-Gly-L-Ser.

Herstellung peroraler Arzneiformen

Die Verbindungen der Formel I können zur peroralen Verabreichung zu Tabletten folgender Zusammensetzung formuliert werden:

| | |
|---|---|
| Peptid-Wirkstoff | 50 - 150 mg |
| Mikrokristalline Cellulose (Avicel®) | 98 mg |
| Lactose, granuliert | 250 - 150 mg |
| Magnesiumstearat | 2 mg |
| Gesamtgewicht | 400 mg |

Der pulverförmige Peptid-Wirkstoff wird im Taumelmischer mit der mikrokristallinen Cellulose vermischt, danach die granulierte Lactose zugemischt und zuletzt das Magnesiumstearat zugegeben und weitere 20 Minuten im Taumelmischer gemischt. Daraus werden in üblicher Weise runde, schwach gewölbte Tabletten gepreßt und diese zum Schutz gegen Magensaft mit folgender Polymerlösung besprüht (berechnet auf 3 kg Tabletten).

| | |
|---|---|
| Methacrylsäure-Methylmethacrylat-1:1 Copolymerisat | 85 g |
| Polyethylenglykol | 15 g |
| Talkum | 50 g |
| Wasser | 50 g |
| Isopropanol | 400 g |
| Aceton | 400 g |
| | 1000 g |

Der Lackauftrag erfolgt in einem üblichen rotierenden Dragierkessel mit einer Luftdruckspritzpistole unter Einblasen von Warmluft von ca. 30 - 50°C innerhalb von 2 Stunden.

Arzneimittel mit diuretischer Wirksamkeit

Patentansprüche

1. Arzneimittel mit diuretischer Wirksamkeit,

dadurch gekennzeichnet,

daß als Wirkstoff ein Peptid der Formel I

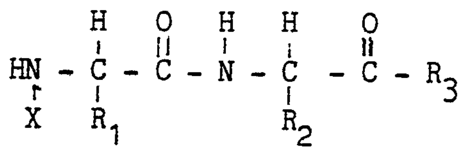

I

worin a) $R_1$ und $R_2$ für Wasserstoff und $R_3$ für -OH oder für einen Rest

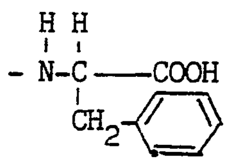

oder einen Rest

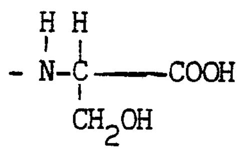

oder einen Rest Y oder

worin b) $R_1$ für Wasserstoff, $R_2$ für $CH_2OH$ oder $-CH_2-$ 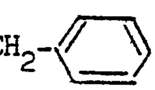 und $R_3$ für -OH oder einen Rest Y oder

worin c) $R_1$ für einen Rest $-CH_2-OH$ und $R_2$ für Wasserstoff oder einen Rest $-CH_2-$ 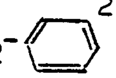 und $R_3$ für -OH oder einen Rest Y oder

worin d) $R_1$ für einen Rest $-CH_2-$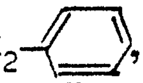, $R_2$ für Wasserstoff oder für einen Rest $-CH_2OH$ und $R_3$ für -OH oder einen Rest $-\overset{H}{N}-CH_2-COOH$ oder einen Rest Y stehen und worin X für Wasserstoff, Methyl, einen Prolylrest oder eine N-Schutzgruppe und Y für $-NH_2$, $-OR_4$, worin $R_4$ für einen gegebenenfalls verzweigten, gegebenenfalls cyclischen Alkylrest mit 1 bis 8 Kohlenstoffatomen, einen Benzyl- oder Phenylrest

oder einen Rest

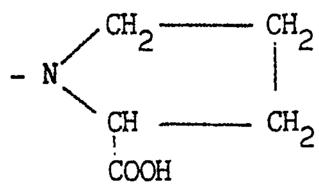

steht,
oder solche Vorstufen, die unter physiologischen Bedingungen Peptide der Formel I bilden

enthalten.

2. Arzneimittel gemäß Anspruch 1, dadurch gekennzeichnet, daß die Wirkstoffe in einer für parenterale Verabreichung geeigneten Verabreichungsform vorliegen.

3. Arzneimittel gemäß den Ansprüchen 1 und 2, dadurch gekennzeichnet, daß als Wirkstoffe der Formel I die Peptide der Formel

| | |
|---|---|
| Gly-Gly-L-Phe | und/oder |
| Gly-Gly | und/oder |
| Gly-Gly-L-Ser | und/oder |

Gly-L-Ser

vorliegen.

4. Arzneimittel gemäß den Ansprüchen 1 und 2, dadurch gekennzeichnet, daß als Wirkstoffe die Peptide

| | |
|---|---|
| L-Phe-Gly-Gly | und/oder |
| L-Phe-Gly | und/oder |
| L-Phe-L-Ser-Gly | und/oder |
| L-Phe-L-Ser | |

enthalten sind.

5. Arzneimittel gemäß den Ansprüchen 1 und 2, dadurch gekennzeichnet, daß als Wirkstoffe die Peptide

| | |
|---|---|
| L-Ser-Gly-Gly | und/oder |
| L-Ser-Gly | und/oder |
| L-Ser-L-Phe-Gly | und/oder |
| L-Ser-L-Phe | |

enthalten sind.

6. Arzneimittel gemäß den Ansprüchen 1 bis 5, dadurch gekennzeichnet, daß die Peptide der Formel I in Tagesdosen von 250 - 2500 µg/kg angewendet werden.

7. Arzneimittel gemäß den Ansprüchen 1 bis 6, dadurch gekennzeichnet, daß sie zusätzlich noch einen oder mehrere

Calcium-antagonistische Wirkstoffe enthalten.

8. Verwendung der Peptide der Formel I gemäß den Ansprüchen 1 bis 5 als Wirkstoffe bei der Therapie von durch Diurese beeinflußbaren Krankheiten.

9. Verwendung der Peptide der Formel I gemäß Anspruch 8 in Mengen von 250 bis 2500 µg/kg.